# EUROPEAN PATENT APPLICATION

(11) **EP 4 570 281 A1**
(43) Date of publication of application: **18.06.2025**
(21) Application number: 24150643.5
(22) Date of filing: 08.01.2024
(51) Int. Cl.: A61M 5/142, A61M 5/20, A61M 5/14, A61M 5/158

(54) **PUMPING AND INSERTING MECHANISM AND RELATED AUTOMATED INJECTION SYSTEM**

(30) Priority: 12.12.2023 US 202318537760
(71) Applicant: Altek Biotechnology Corporation, 300 Hsinchu City (TW)
(72) Inventor: Chu, Yi, 300 Hsinchu City (TW); Huang, Yu-Cheng, 300 Hsinchu City (TW)
(74) Representative: Straus, Alexander

(57) **Abstract**

A pumping and inserting mechanism(13A) is provided and includes a pumping assembly(131A,131B,131C,131D,131E,131F,131G,131H,131I), a driving assembly(132A,132B,132E,132F,132G,132H,132I) and an inserting assembly(133A,133E,133F). The pumping assembly(131A,131B,131C,131D,131E,131F,131G,131H,131I) includes an input portion(1313A,1313B,1313G,1313H,1313I) and an output portion(1314A,1314B,1314C,1314D). The driving assembly(132A,132B,132E,132F,132G,132H,132I) is coupled to the input portion(1313A,1313B,1313G,1313H,1313I) of the pumping assembly(131A,131B,131C,131D,131E,131F,131G,131H,131I) and configured to drive a rotating movement of the input portion(1313A,1313B,1313G,1313H,1313I) of the pumping assembly(131A,131B,131C,131D,131E,131F,131G,131H,131I) to drive a rotating movement of the output portion(1314A,1314B,1314C,1314D) of the pumping assembly(131A,131B,131C,131D,131E,131F,131G,131H,131I). The inserting assembly(133A,133E,133F) includes a triggering component(1331A,1331B,1331C,1331D) and an inserting component(1332A,1332B,1332C,1332D). The triggering component(1331A,1331B,1331C,1331D) is driven to rotate to trigger an inserting movement of the inserting component(1332A,1332B,1332C,1332D) in response to the rotating movement of the output portion(1314A,1314B,1314C,1314D) of the pumping assembly(131A,131B,131C,131D,131E,131F,131G,131H,131I). Besides, a related automated injection system is also provided.

## Description

### Field of the Invention

The present invention relates to a pumping and inserting mechanism and a related automated injection system according to the pre-characterizing clauses of claims 1 and 11.

### Background of the Invention

An automated injection system is designed for drug delivery. However, the conventional automated injection systems available on the market are unable to meet requirements of simple structure, compact size and reusability, especially for large volume delivery. Therefore, an improvement of the automated injection system is urgently needed.

### Summary of the Invention

This in mind, the present invention aims at providing a pumping and inserting mechanism and a related automated injection system for addressing the aforementioned needs.

This is achieved by a pumping and inserting mechanism and a related automated injection system according to claims 1 and 11. The dependent claims pertain to corresponding further developments and improvements.

As will be seen more clearly from the detailed description following below, the claimed pumping and inserting mechanism includes a pumping assembly, a driving assembly and an inserting assembly. The pumping assembly includes an input portion and an output portion. The driving assembly is coupled to the input portion of the pumping assembly and configured to drive a rotating movement of the input portion of the pumping assembly to drive a rotating movement of the output portion of the pumping assembly. The inserting assembly includes a triggering component and an inserting component. The triggering component is driven to rotate to trigger an inserting movement of the inserting component in response to the rotating movement of the output portion of the pumping assembly.

Besides, the claimed automated injection system includes a case, a reservoir and the aforementioned pumping and inserting mechanism. The reservoir is mounted on the case internally or externally, or separated from the case. The pumping assembly is at least partially mounted inside the case. An inlet of the pumping assembly is communicated with the reservoir. The driving assembly is mounted on the case internally or externally. The inserting assembly is at least partially mounted inside the case. The inserting component is communicated with an outlet of the pumping assembly.

In summary, the present invention is configured to drive the pumping assembly to deliver drug and further to trigger the inserting movement of the inserting component with the same driving assembly. Therefore, the present invention has simple structure and compact size. Besides, the driving assembly can be optionally detached from the pumping assembly and used as a reusable unit. Therefore, the present invention also has reusability.

These and other objectives of the present invention will no doubt become obvious to those of ordinary skill in the art after reading the following detailed description of the preferred embodiment that is illustrated in the various figures and drawings.

### Brief Description of the Drawings

In the following, the invention is further illustrated by way of example, taking reference to the accompanying drawings. Thereof
FIG. 1 is a diagram of an automated injection system according to a first embodiment of the present invention,
FIG. 2 and FIG. 3 are partial internal structural diagrams of the automated injection system at different states according to the first embodiment of the present invention,
FIG. 4 is a partial exploded diagram of an input portion of a pumping assembly, an adaptor and an output shaft of a driving component according to the first embodiment of the present invention,
FIG. 5 is a partial diagram of the pumping assembly according to the first embodiment of the present invention,
FIG. 6 is a partial exploded diagram of an output portion of a pumping assembly, an engaging component and a triggering component according to the first embodiment of the present invention,
FIG. 7 is a partial diagram illustrating the triggering component triggers an inserting movement of an inserting component according to the first embodiment of the present invention,
FIG. 8 is a partial diagram illustrating a triggering component triggers an inserting movement of an inserting component of an automated injection system according to a second embodiment of the present invention,
FIG. 9 is a partial diagram illustrating a triggering component triggers an inserting movement of an inserting component of an automated injection system according to a third embodiment of the present invention,
FIG. 10 is a partial diagram illustrating a triggering component triggers an inserting movement of an inserting component of an automated injection system according to a fourth embodiment of the present invention,
FIG. 11 is a partial diagram of an automated injection system according to a fifth embodiment of the present invention,
FIG. 12 is a partial diagram of an automated injection system according to a sixth embodiment of the present invention,
FIG. 13 is a partial diagram of an automated injection system according to a seventh embodiment of the present invention,
FIG. 14 is a partial diagram of an automated injection system according to an eighth embodiment of the present invention, and
FIG. 15 is a partial diagram of an automated injection system according to a ninth embodiment of the present invention.

### Detailed Description

In the following detailed description of the preferred embodiments, reference is made to the accompanying drawings which form a part hereof, and in which is shown by way of illustration specific embodiments in which the invention may be practiced. In this regard, directional terminology, such as "top", "bottom", "left", "right", "front", "back", etc., is used with reference to the orientation of the Figure(s) being described. The components of the present invention can be positioned in a number of different orientations. As such, the directional terminology is used for purposes of illustration and is in no way limiting. Accordingly, the drawings and descriptions will be regarded as illustrative in nature and not as restrictive. Also, if not specified, the term "couple" is intended to mean either an indirect or direct electrical/mechanical connection. Thus, if a first device is connected or coupled to a second device, that connection may be through a direct electrical/mechanical connection, or through an indirect electrical/mechanical connection via other devices and connections.

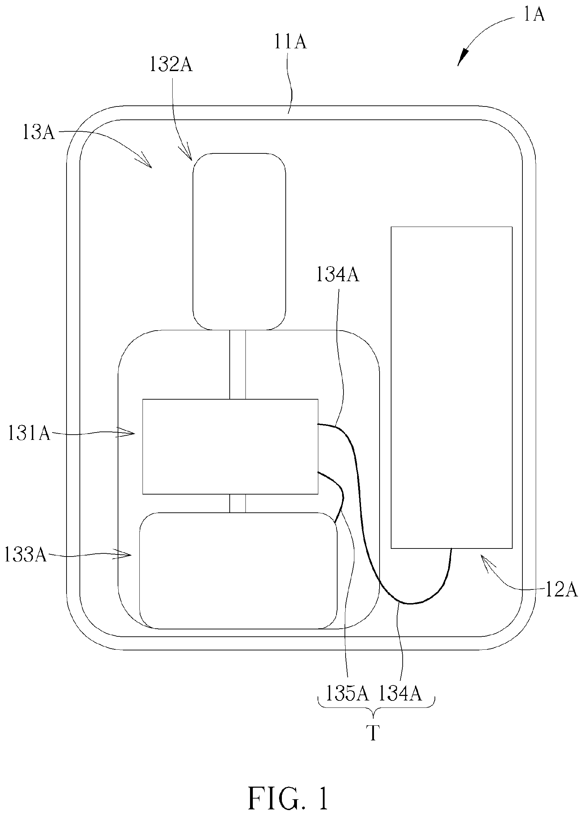

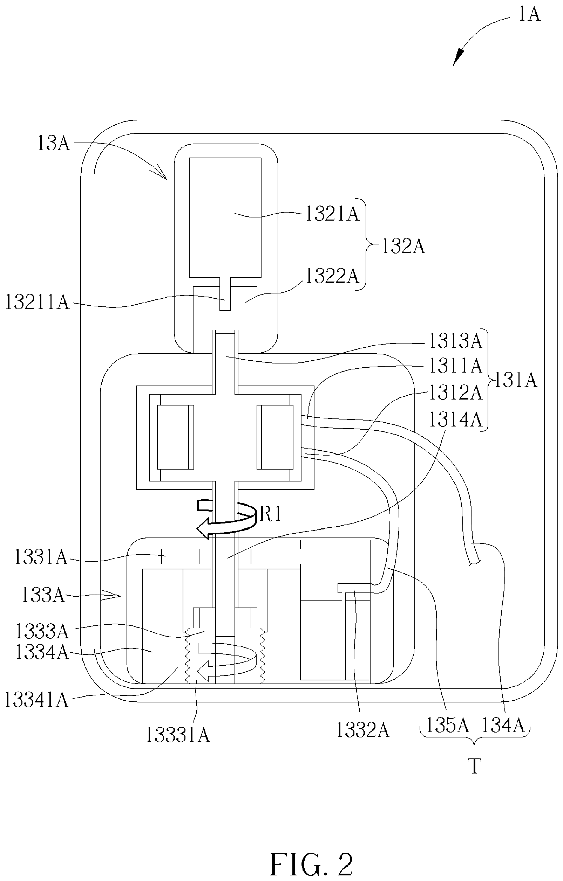

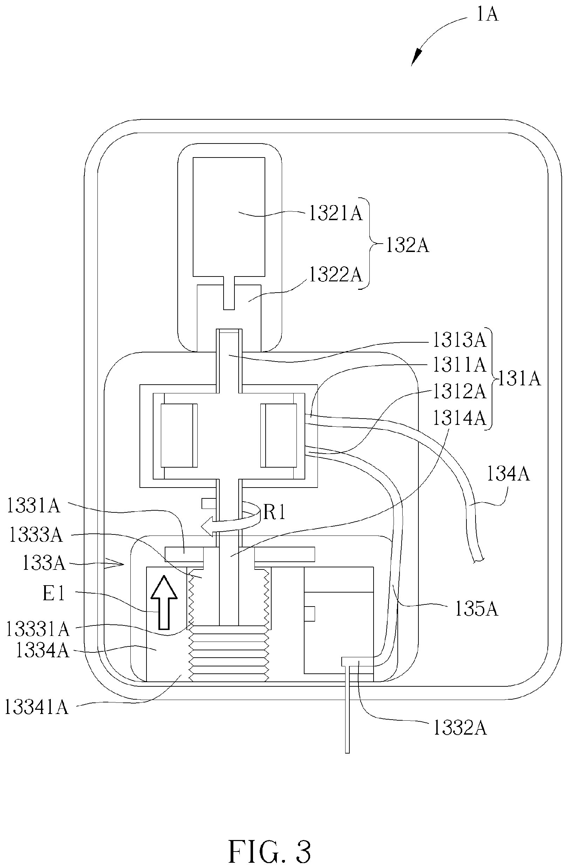

Please refer to FIG. 1 to FIG. 3. FIG. 1 is a diagram of an automated injection system 1A according to a first embodiment of the present invention. FIG. 2 and FIG. 3 are partial internal structural diagrams of the automated injection system 1A at different states according to the first embodiment of the present invention. As shown in FIG. 1 to FIG. 3, the automated injection system 1A includes a case 11A, a reservoir 12A and a pumping and inserting mechanism 13A. The reservoir 12A is mounted inside the case 11A. The pumping and inserting mechanism 13A includes a pumping assembly 131A, a driving assembly 132A and an inserting assembly 133A. The pumping assembly 131A is mounted inside the case 11A and includes an inlet 1311A, an outlet 1312A, an input portion 1313A and an output portion 1314A. The inlet 1311A of the pumping assembly 131A is communicated with the reservoir 12A, e.g., by an inlet portion134A of a tube T. The driving assembly 132A is mounted inside the case 11A and coupled to, e.g., directly connected to, the input portion 1313A of the pumping assembly 131A and configured to drive a rotating movement of the input portion 1313A of the pumping assembly 131A along a first rotating direction R1 to drive a rotating movement of the output portion 1314A of the pumping assembly 131A along the first rotating direction R1. The inserting assembly 133A is mounted inside the case 11A and includes a triggering component 1331A and an inserting component 1332A. The inserting component 1332A is communicated with the outlet 1312A of the pumping assembly 131A, e.g., by an outlet portion 135A of the tube T. Specifically, the inserting component 1332A can include a hollow needle for skin penetration and drug delivery. The triggering component 1331A is driven to rotate along the first rotating direction R1 to trigger an inserting movement of the inserting component 1332A in response to the rotating movement of the output portion 1314A of the pumping assembly 131A along the first rotating direction R1. Besides, in the first embodiment, the pumping assembly 131A and the inserting assembly 133A can be combined together as an integrated unit separated from the driving assembly 132A.

However, the present invention is not limited to this embodiment. It depends on practical demands. For example, in another embodiment, the reservoir can be mounted on the case externally or separated from the case. Alternatively, in another embodiment, the driving assembly can be mounted on the case externally, and the input portion of the pumping assembly can be exposed out of the case and connected to the driving assembly. Alternatively, in another embodiment, a portion of the inserting assembly can be exposed out of the case. Alternatively, in another embodiment, the driving assembly, the pumping assembly and the inserting assembly can be combined together as an integrated unit.

As shown in FIG. 2 and FIG. 3, the driving assembly 132A includes a driving component 1321A and an adaptor 1322A, and the adaptor 1322A is mounted between and coupled to, e.g., directly connected to, the driving component 1321A and the input portion 1313A of the pumping assembly 131A and detachable from the input portion 1313A of the pumping assembly 131A.

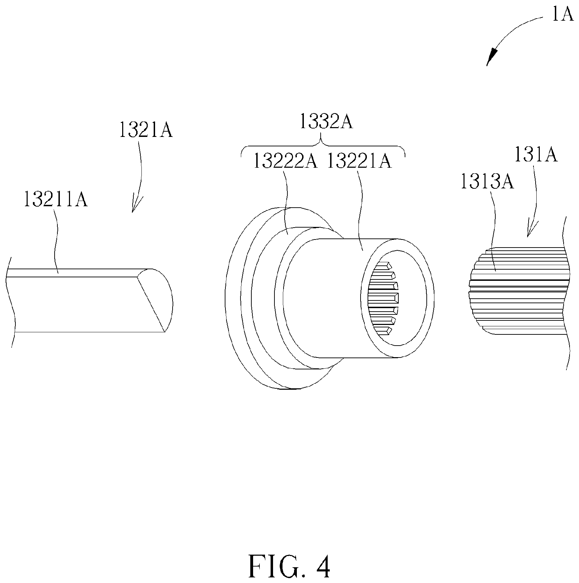

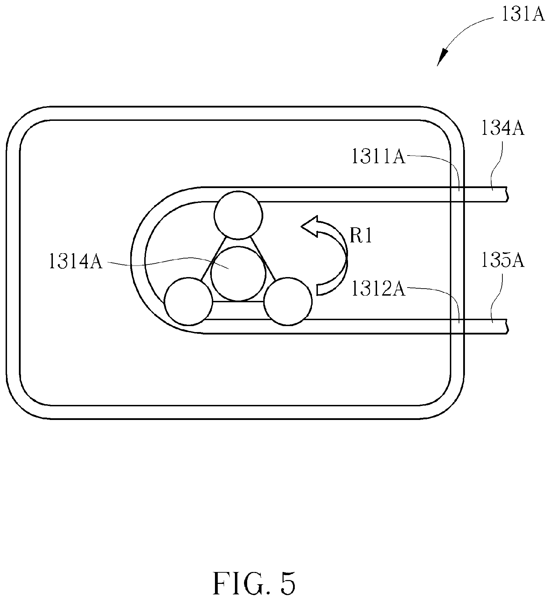

Please refer to FIG. 2, FIG. 3, FIG. 4 and FIG. 5. FIG. 4 is a partial exploded diagram of the input portion 1313A of the pumping assembly 131A, the adaptor 1322A and an output shaft 13211A of the driving component 1321A according to the first embodiment of the present invention. FIG. 5 is a partial diagram of the pumping assembly 131A according to the first embodiment of the present invention. Specifically, as shown in FIG. 2, FIG. 3, FIG. 4 and FIG. 5, the driving component 1321A can be an electric motor, and the pumping assembly 131A can be a peristaltic pump. Furthermore, the adaptor 1322A can have a first sleeve section 13221A and a second sleeve portion 13222A, and the input portion 1313A of the pumping assembly 131A and the output shaft 13211A of the driving component 1321A are at least partially inserted into the first sleeve section 13221A and the second sleeve portion 13222A respectively. More specifically, the output shaft 13211A of the driving component 1321A can be a D-shaped shaft of the driving component 1321A, and the input portion 1313A of the pumping assembly 131A can be an input end of a spline shaft of the pumping assembly 131A. Besides, the first sleeve section 13221A and the second sleeve section 13222A can respectively be a spline sleeve section and a D-shaped sleeve section. Understandably, the shapes of the output shaft of the driving component and the input portion of the pumping assembly are not limited to this embodiment. It depends on practical demands.

Besides, as shown in FIG. 2 and FIG. 3, the inserting assembly 133A further includes an engaging component 1333A coupled to, e.g., directly connected to, the output portion 1314A of the pumping assembly 131A. The engaging component 1333A is configured to move from a disengaged state as shown in FIG. 2 to an engaged state as shown in FIG. 3 in response to the rotating movement of the input portion 1313A of the pumping assembly 131A along the first rotating direction R1. Specifically, the output portion 1314A of the pumping assembly 131A can be an output end of the spline shaft of the pumping assembly 131A and at least partially inserted into the engaging component 1333A, such that the engaging component 1333A can be driven by the output portion 1314A of the pumping assembly 131A to move from the disengaged state as shown in FIG. 2 to the engaged state as shown in FIG. 3 in response to the rotating movement of the input portion 1313A of the pumping assembly 131A along the first rotating direction R1.

As shown in FIG. 2, the engaging component 1333A is disengaged from the triggering component 1331A for not driving the triggering component 1331A to rotate when the engaging component 1333A is not in the engaged state. As shown in FIG. 3, the engaging component 1333A is engaged with the triggering component 1331A for driving the triggering component 1331A to rotate together with the engaging component 1333A along the first rotating direction R1 when the engaging component 1333A is in the engaged state. By such configuration, the driving assembly 132A can operate the pumping assembly 131A to work before the inserting assembly 133A, so as to discharge air inside the tube T communicated between the reservoir 12A and the inserting component 1332A, e.g., the inlet portion 134A and the outlet portion 135A of the tube T, and/or to discharge air inside a channel of the inserting component 1332A before operation of the inserting movement of the inserting component 1332A, and then the driving assembly 132A can drive the triggering component 1331A to trigger the inserting movement of the inserting component 1332A and drive the pumping assembly 131A to deliver drug, which reduces a potential risk caused by injecting an excessive amount of air into a body.

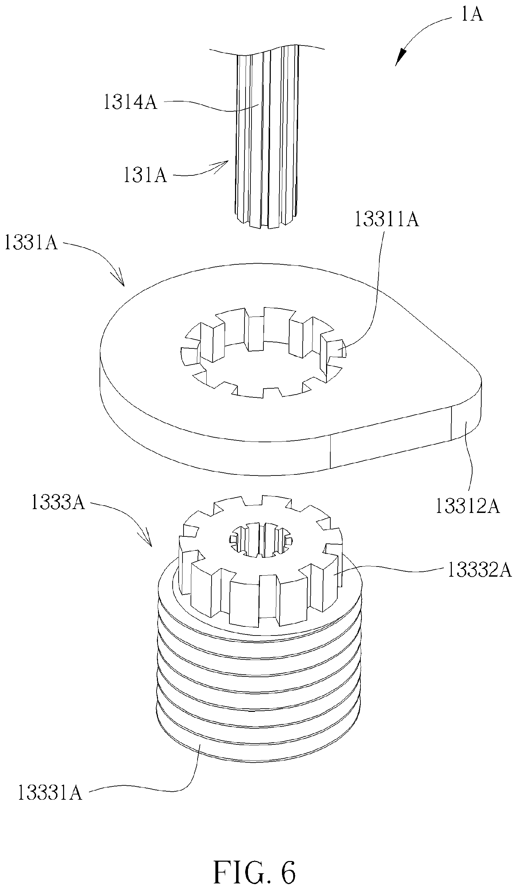

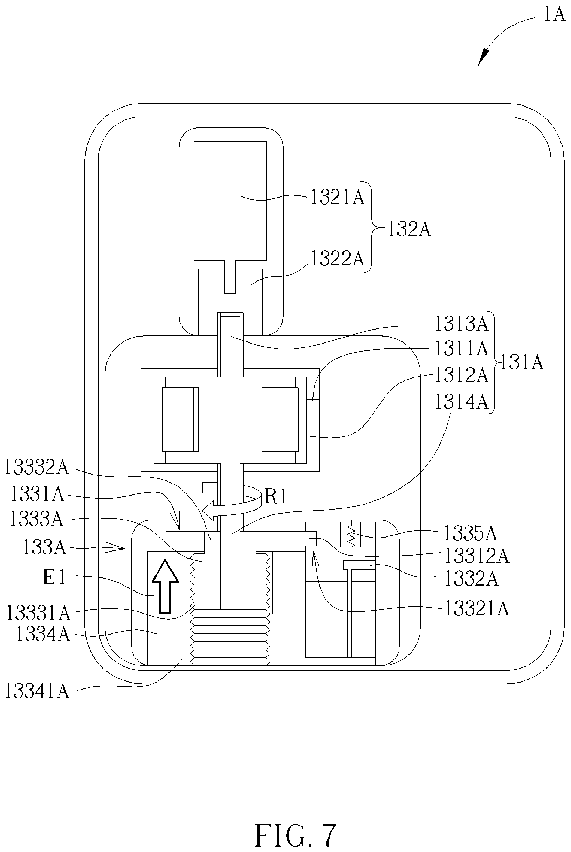

Please refer to FIG. 2, FIG. 3, FIG. 6 and FIG. 7. FIG. 6 is a partial exploded diagram of the output portion 1314A of the pumping assembly 131A, the engaging component 1333A and the triggering component 1331A according to the first embodiment of the present invention. FIG. 7 is a partial diagram illustrating the triggering component 1331A triggers the inserting movement of the inserting component 1332A according to the first embodiment of the present invention. Specifically, as shown in FIG. 2, FIG. 3, FIG. 6 and FIG. 7, a rotating axis of the triggering component 1331A can be coincided with a rotating axis of the output portion 1314A of the pumping assembly 131A, a rotating axis of the input portion 1313A of the pumping assembly 131A and a rotating axis of the engaging component 1333A. Furthermore, the engaging component 1333A can be a rotatable component and rotatable relative to a fixed component 1334A of the inserting assembly 133A. The output portion 1314A of the pumping assembly 131A can be disposed through the triggering component 1331A and at least partially inserted into the engaging component 1333A. More specifically, the engaging component 1333A includes a first cooperating structure 13331A, e.g., an external threaded structure. The fixed component 1334A of the inserting assembly 133A includes a second cooperating structure 13341A, e.g., an internal threaded structure, configured to cooperate with the first cooperating structure 13331A to drive the engaging component 1333A to move from the disengaged state as shown in FIG. 2 to the engaged state as shown in FIG. 3 when the engaging component 1333A is driven to rotate along the first rotating direction R1 in response to the rotating movement of the output portion 1314A of the pumping assembly 131A along the first rotating direction R1, such that the engaging component 1333A can be moved along an extending direction of the output portion 1314A of the pumping assembly 131A, e.g., an arrow direction E1, by a cooperation of the first cooperating structure 13331A and the second cooperating structure 13341A when the engaging component 1333A rotates along the first rotating direction R1.

Besides, as shown in FIG. 6 and FIG. 7, the triggering component 1331A includes a first engaging structure 13311A. The output portion 1314A of the pumping assembly 131A is disposed through the first engaging structure, and the engaging component 1333A includes a second engaging structure 13332A configured to engage with the first engaging structure 13311A to allow the engaging component 1333A to drive the triggering component 1331A to rotate together with the engaging component 1333A along the first rotating direction R1 due to an engagement of the first engaging structure 13311A and the second engaging structure 13332A when the engaging component 1333A moves along the extending direction to a predetermined position as shown in FIG. 3. Besides, the first cooperating structure 13331A of the engaging component 1333A and the second cooperating structure 13341A of the fixed component 1334A are disengaged from each other when the engaging component 1333A moves along the extending direction to the predetermined position as shown in FIG. 3.

In addition, in the first embodiment, as shown in FIG. 7, the inserting component 1332A can be biased by a resilient component 1335A, e.g., a compression spring, and a protrusion 13312A of the triggering component 1331A can engage with an engaging notch 13321A formed on the inserting component 1332A. When the protrusion 13312A of the triggering component 1331A engages with the engaging notch 13321A, the inserting component 1332A is locked and restrained from moving by an engagement of the protrusion 13312A of the triggering component 1331A and the engaging notch 13321A. When the triggering component 1331A is driven by the engaging component 1333A engaged with triggering component 1331A to disengage the protrusion 13312A of the engaging component 1333A from the engaging notch 13321A, the inserting component 1332A is unlocked, such that the resilient component 1335A can drive the inserting movement of the inserting component 1332A.

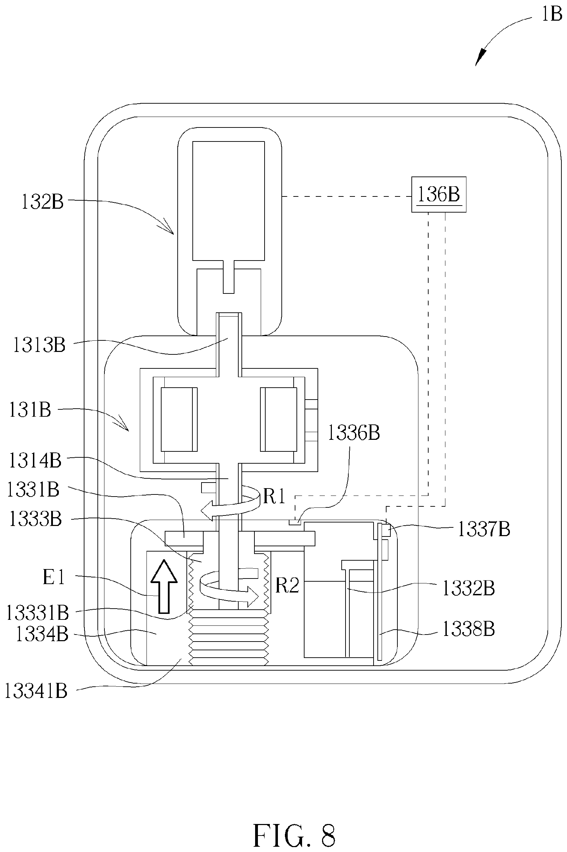

Please refer to FIG. 8. FIG. 8 is a partial diagram of illustrating a triggering component 1331B triggers an inserting movement of an inserting component 1332B of an automated injection system 1B according to a second embodiment of the present invention. The automated injection system 1B of the second embodiment is similar to the automated injection system 1A of the first embodiment. As shown in FIG. 8, different from the first embodiment, in the second embodiment, a driving assembly 132B can be controlled by a control unit 136B, e.g., a processor or a circuit, to drive an input portion 1313B of a pumping assembly 131B to rotate along the first rotating direction R1 to drive an output portion 1314B of the pumping assembly 131B to rotate along the first rotating direction R1 for discharging air, triggering the inserting movement of the inserting component 1332B and delivering drug, and then further drive the input portion 1313B of the pumping assembly 131B to rotate along a second rotating direction R2 opposite to the first rotating direction R1 to drive the output portion 1314B of the pumping assembly 131B to rotate along the second rotating direction R2 for triggering an retracting movement of the inserting component 1332B.

It should be noticed that a first cooperating structure 13331B of an engaging component 1333B and a second cooperating structure 13341B of a fixed component 1334B are disengaged from each other when the engaging component 1333B moves along the arrow direction E1 to the predetermined position as shown in FIG. 8. Therefore, the engaging component 1333B cannot be driven to move along a direction opposite to the arrow direction E1 when the output portion 1314B of the pumping assembly 131B rotates along the second rotating direction R2.

Furthermore, in the second embodiment, the triggering component 1331B can cooperate with a sensing device, which can include at least one light sensor 1336B, electrically coupled to, e.g., directly connected to, the control unit 136B, and the inserting component 1332B can be driven by an insertion and retraction driving device, which can include a motor 1337B electrically coupled to, e.g., directly connected to, the control unit 136B and a screw rod 1338B driven by the motor 1337B, in response to a sensing result of the sensing device.

When the sensing device detects the triggering component 1331B rotates along the first rotating direction R1, the insertion and retraction driving device can drive the inserting component 1332B to move along an inserting direction, e.g., by rotating the screw rod 1338B along a clockwise direction, and when the sensing device detects the triggering component 1331B rotates along the second rotating direction R2, the insertion and retraction driving device can drive the inserting component 1332B to move along a retracting direction, e.g., by rotating the screw rod 1338B along a counterclockwise direction.

Understandably, in another embodiment, the driving assembly can be configured to drive the input portion of the pumping assembly to rotate along the first rotating direction to drive the output portion of the pumping assembly to rotate along the first rotating direction and then stop rotating, and the insertion and retraction driving device can be configured to drive the inserting component to move along the inserting direction when the sensing device detects the triggering component rotates along the first rotating direction and further to drive the inserting component to move along the retracting direction by rotating the screw rod along the second rotating direction when the sensing device detects the triggering component stops rotating.

Besides, in the second embodiment, the controller 136B can be further electrically coupled to, e.g., directly connected to, a detector, e.g., a liquid level sensor or a timer or a counter, so as to determine the drug injection is completed and to switch a rotating direction of the output portion 1314B of the pumping assembly 131B from the first rotating direction R1 to the second rotating direction R2 for triggering the insertion and retraction driving device to drive the inserting component 1332B to move along the retracting direction.

Other details of the second embodiment are the same as the ones of the first embodiment. Detailed description is omitted herein for simplicity.

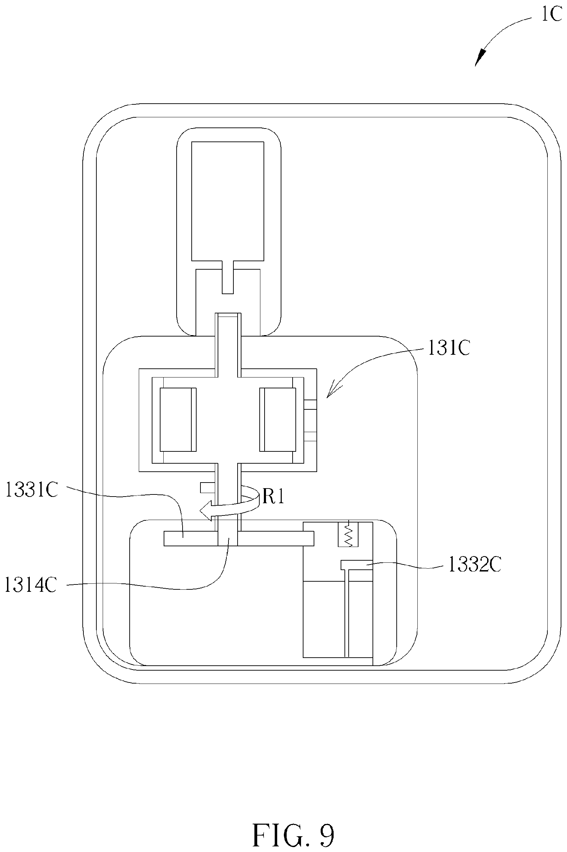

Please refer to FIG. 9. FIG. 9 is a partial diagram illustrating a triggering component 1331C triggers an inserting movement of an inserting component 1332C of an automated injection system 1C according to a third embodiment of the present invention. The automated injection system 1C of the third embodiment is similar to the automated injection system 1A of the first embodiment. As shown in FIG. 9, different from the first embodiment, in the third embodiment, the engaging component is omitted, and an output portion 1314C of a pumping assembly 131C is coupled to, e.g., directly connected to, the triggering component 1331C directly. The automated injection system 1C is suitable for a condition of that air discharge before insertion of the inserting component 1332 is not necessary. Other details of the third embodiment are the same as the ones of the first embodiment. Detailed description is omitted herein for simplicity.

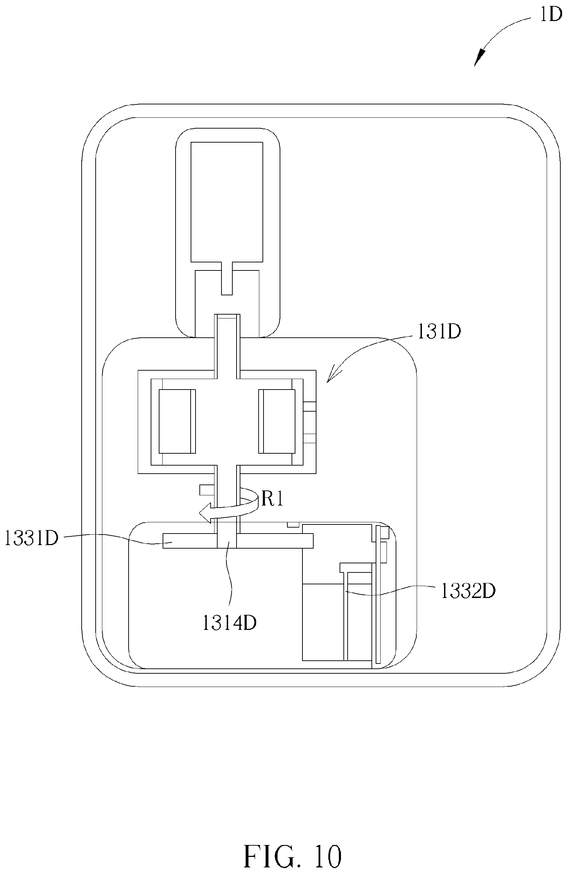

Please refer to FIG. 10. FIG. 10 is a partial diagram illustrating a triggering component 1331D triggers an inserting movement of an inserting component 1332D of an automated injection system 1D according to a fourth embodiment of the present invention. The automated injection system 1D of the fourth embodiment is similar to the automated injection system 1B of the second embodiment. As shown in FIG. 10, different from the second embodiment, in the fourth embodiment, the engaging component is omitted, and an output portion 1314D of a pumping assembly 131D is coupled to, e.g., directly connected to, the triggering component 1331D directly. The automated injection system 1D is also suitable for a condition of that air discharge is not necessary. Other details of the fourth embodiment are to the same as the ones of the second embodiment. Detailed description is omitted herein for simplicity.

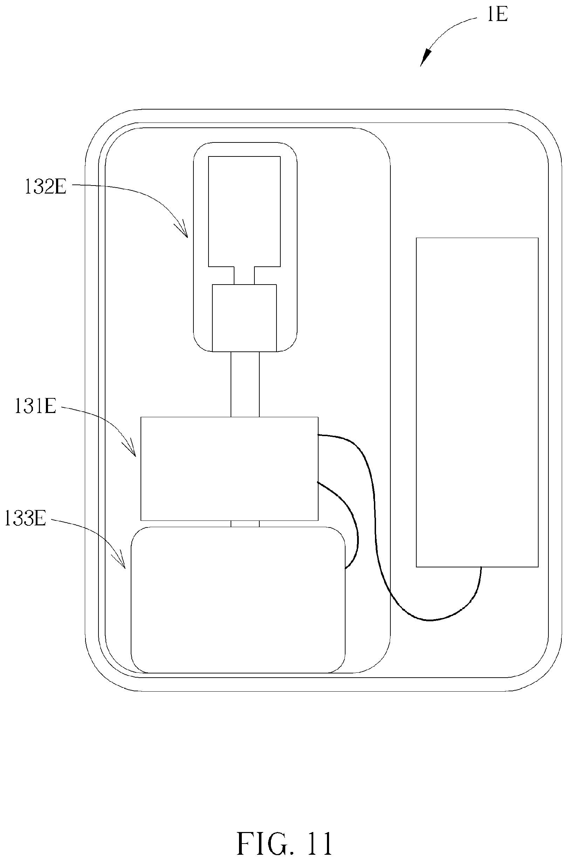

Please refer to FIG. 11. FIG. 11 is a partial diagram of an automated injection system 1E according to a fifth embodiment of the present invention. The automated injection system 1E of the fifth embodiment is similar to the automated injection system 1A of the first embodiment. As shown in FIG. 11, different from the first embodiment, in the fifth embodiment, a pumping assembly 131E, a driving assembly 132E and an inserting assembly 133E are combined together as an integrated unit. Other details of the fifth embodiment are the same as the ones of the first embodiment. Detailed description is omitted herein for simplicity. Understandably, in a similar embodiment, the driving assembly and the inserting assembly also can be the same as the ones of the second embodiment to enable the inserting component to retract after completion of drug delivery.

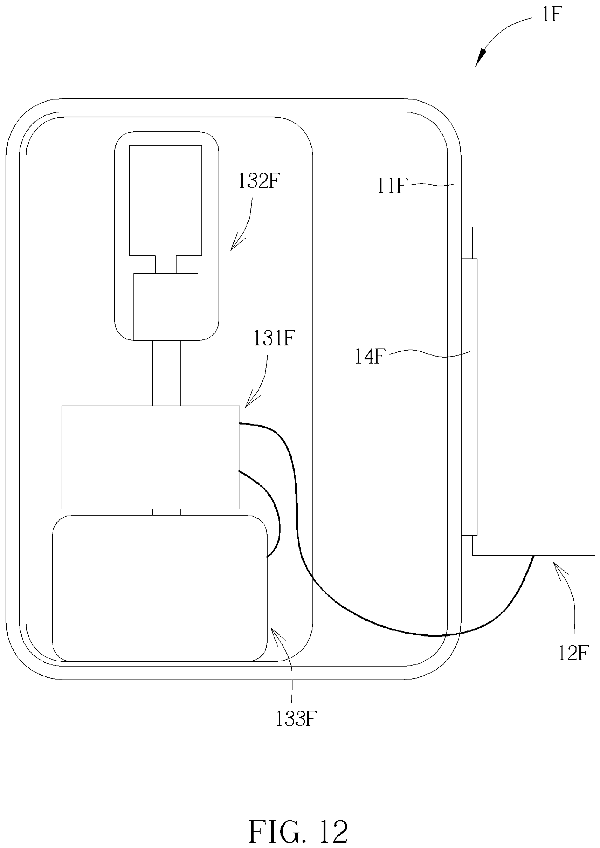

Please refer to FIG. 12. FIG. 12 is a partial diagram of an automated injection system 1F according to a sixth embodiment of the present invention. The automated injection system 1F of the sixth embodiment is similar to the automated injection system 1A of the first embodiment. As shown in FIG. 12, different from the first embodiment, in the sixth embodiment, a pumping assembly 131F, a driving assembly 132F and an inserting assembly 133F are combined together as an integrated unit. Besides, in the sixth embodiment, a reservoir 12F is mounted on a case 11F externally, e.g., by a mounting rail 14F. Understandably, in a similar embodiment, the reservoir can be separated from the case and held by a stand or any other holder. Other details of the sixth embodiment are the same as the ones of the first embodiment. Detailed description is omitted herein for simplicity. Understandably, in a similar embodiment, the driving assembly and the inserting assembly also can be the same as the ones of the second embodiment to enable the inserting component to retract after completion of drug delivery.

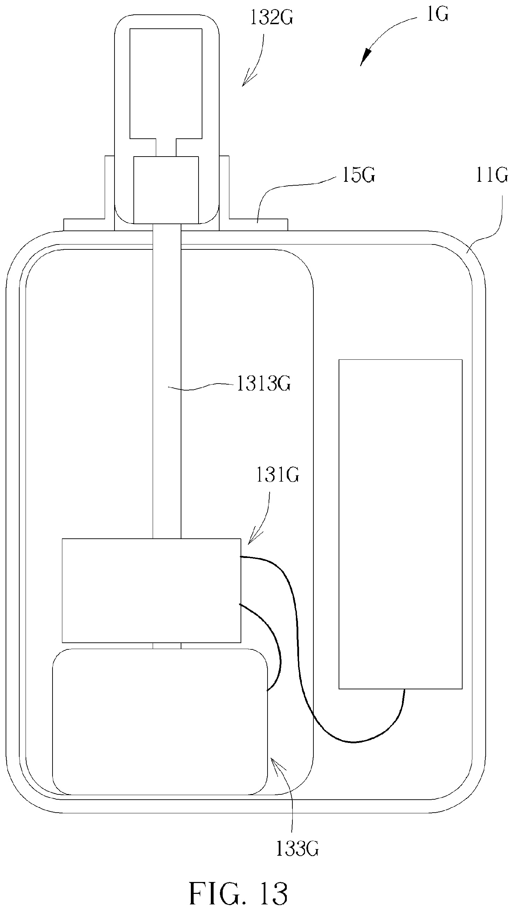

Please refer to FIG. 13. FIG. 13 is a partial diagram of an automated injection system 1G according to a seventh embodiment of the present invention. The automated injection system 1G of the seventh embodiment is similar to the automated injection system 1A of the first embodiment. As shown in FIG. 13, different from the first embodiment, in the seventh embodiment, a driving assembly 132G is mounted on a case 11G externally, e.g., by a bracket 15G, and an input portion 1313G of a pumping assembly 131G is exposed out of the case 11G and coupled to, e.g., directly connected to, the driving assembly 132G. Other details of the seventh embodiment are the same as the ones of the first embodiment. Detailed description is omitted herein for simplicity. Understandably, in a similar embodiment, the driving assembly and the inserting assembly also can be the same as the ones of the second embodiment to enable the inserting component to retract after completion of drug delivery.

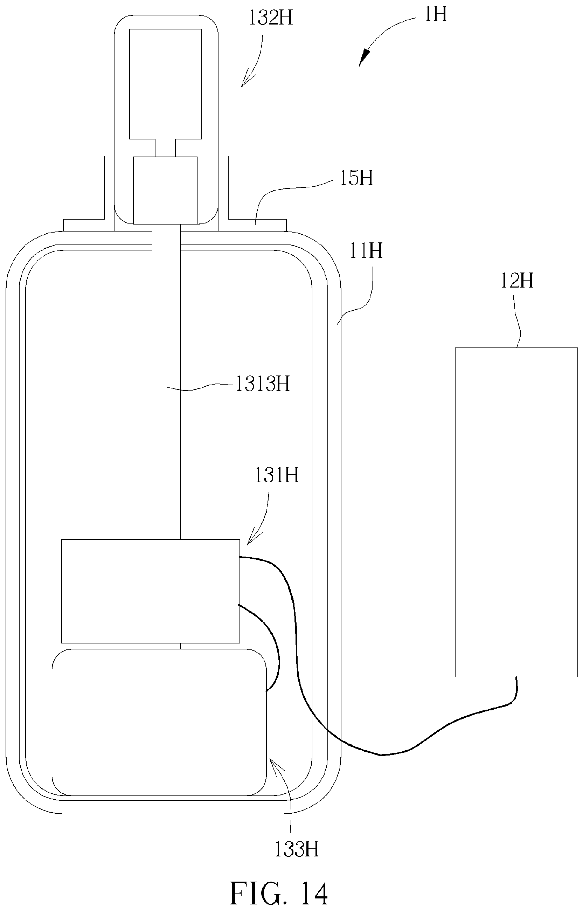

Please refer to FIG. 14. FIG. 14 is a partial diagram of an automated injection system 1H according to an eighth embodiment of the present invention. The automated injection system 1H of the eighth embodiment is similar to the automated injection system 1A of the first embodiment. As shown in FIG. 14, different from the first embodiment, in the eighth embodiment, a driving assembly 132H is mounted on a case 11H externally, e.g., by a bracket 15H, and an input portion 1313H of a pumping assembly 131H is exposed out of the case 11H and coupled to, e.g., directly connected to, the driving assembly 132H. Besides, a reservoir 12H is separated from the case 11H and can be held by an additional holder, e.g., a drip stand, which is not shown in the figure. Other details of the eighth embodiment are the same as the ones of the first embodiment. Detailed description is omitted herein for simplicity. Understandably, in a similar embodiment, the driving assembly and the inserting assembly also can be the same as the ones of the second embodiment to enable the inserting component to retract after completion of drug delivery.

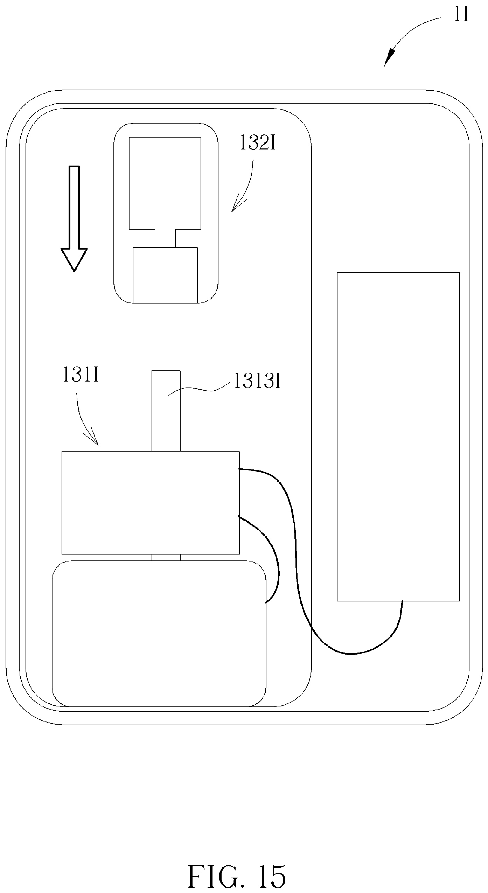

Please refer to FIG. 15. FIG. 15 is a partial diagram of an automated injection system 1I according to a ninth embodiment of the present invention. The automated injection system 1I of the ninth embodiment is similar to the automated injection system 1A of the first embodiment. As shown in FIG. 15, different from the first embodiment, in the ninth embodiment, a driving assembly 132I is configured to move to engage with an input portion 1313I of a pumping assembly 1311, and the driving assembly 132I can be driven by a step motor automatically or by a sliding button or a pressing button manually. Other details of the ninth embodiment are the same as the ones of the first embodiment. Detailed description is omitted herein for simplicity. Understandably, in a similar embodiment, the driving assembly and the inserting assembly also can be the same as the ones of the second embodiment to enable the inserting component to retract after completion of drug delivery.

In contrast to the prior art, the present invention is configured to drive the pumping assembly to deliver drug and further to trigger the inserting movement of the inserting component with the same driving assembly. Therefore, the present invention has simple structure and compact size. Besides, the driving assembly can be optionally detached from the pumping assembly and used as a reusable unit. Therefore, the present invention also has reusability.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. A pumping and inserting mechanism (13A) **characterized by**:
a pumping assembly (131A, 131B, 131C, 131D, 131E, 131F, 131G, 131H, 131I) comprising an input portion (1313A, 1313B, 1313G, 1313H, 1313I) and an output portion (1314A, 1314B, 1314C, 1314D);
a driving assembly (132A, 132B, 132E, 132F, 132G, 132H, 132I) coupled to the input portion (1313A, 1313B, 1313G, 1313H, 1313I) of the pumping assembly (131A, 131B, 131C, 131D, 131E, 131F, 131G, 131H, 131I) and configured to drive a rotating movement of the input portion (1313A, 1313B, 1313G, 1313H, 1313I) of the pumping assembly (131A, 131B, 131C, 131D, 131E, 131F, 131G, 131H, 131I) to drive a rotating movement of the output portion (1314A, 1314B, 1314C, 1314D) of the pumping assembly (131A, 131B, 131C, 131D, 131E, 131F, 131G, 131H, 131I); and
an inserting assembly (133A, 133E, 133F) comprising a triggering component (1331A, 1331B, 1331C, 1331D) and an inserting component (1332A, 1332B, 1332C, 1332D), the triggering component (1331A, 1331B, 1331C, 1331D) being driven to rotate to trigger an inserting movement of the inserting component (1332A, 1332B, 1332C, 1332D) in response to the rotating movement of the output portion (1314A, 1314B, 1314C, 1314D) of the pumping assembly (131A, 131B, 131C, 131D, 131E, 131F, 131G, 131H, 1311).

2. The pumping and inserting mechanism (13A) of claim 1, **characterized in that** the driving assembly (132A, 132B, 132E, 132F, 132G, 132H, 132I) comprises a driving component (1321A) and an adaptor (1322A), and the adaptor (1322A) is mounted between the driving component (1321A) and the input portion (1313A, 1313B, 1313G, 1313H, 1313I) of the pumping assembly (131A, 131B, 131C, 131D, 131E, 131F, 131G, 131H, 131I) and detachable from the input portion (1313A, 1313B, 1313G, 1313H, 1313I) of the pumping assembly (131A, 131B, 131C, 131D, 131E, 131F, 131G, 131H, 1311).

3. The pumping and inserting mechanism (13A) any of claims 1 and 2, further **characterized by** an engaging component (1333A, 1333B) coupled to the output portion (1314A, 1314B) of the pumping assembly (131A, 131B, 131E, 131F, 131G, 131H, 131I) and configured to move from a disengaged state to an engaged state in response to the rotating movement of the input portion (1313A, 1313B, 1313G, 1313H, 1313I) of the pumping assembly (131A, 131B, 131E, 131F, 131G, 131H, 131I), the engaging component (1333A, 1333B) being disengaged from the triggering component (1331A, 1331B) for not driving the triggering component (1331A, 1331B) to rotate when the engaging component (1333A, 1333B) is not in the engaged state, and the engaging component (1333A, 1333B) being engaged with the triggering component (1331A, 1331B) for driving the triggering component (1331A, 1331B) to rotate when the engaging component (1333A, 1333B) is in the engaged state.

4. The pumping and inserting mechanism (13A) of claim 3, **characterized in that** the engaging component (1333A, 1333B) is a rotatable component.

5. The pumping and inserting mechanism (13A) of any of claims 3 and 4, **characterized in that** the engaging component (1333A, 1333B) is rotatable relative to the inserting assembly (133A, 133E, 133F), the engaging component (1333A, 1333B) comprises a first cooperating structure, the inserting assembly (133A, 133E, 133F) comprises a second cooperating structure configured to cooperate with the first cooperating structure to drive the engaging component (1333A, 1333B) to move from the disengaged state to the engaged state when the engaging component (1333A, 1333B) is driven to rotate in response to the rotating movement of the output portion (1314A, 1314B) of the pumping assembly (131A, 131B, 131E, 131F, 131G, 131H, 1311).

6. The pumping and inserting mechanism (13A) of any of claims 3-6, **characterized in that** the output portion (1314A, 1314B) of the pumping assembly (131A, 131B, 131E, 131F, 131G, 131H, 131I) is disposed through the triggering component (1331A, 1331B) and at least partially inserted into the engaging component (1333A, 1333B), and the engaging component (1333A, 1333B) is movable along an extending direction of the output portion (1314A, 1314B) of the pumping assembly (131A, 131B, 131E, 131F, 131G, 131H, 1311) when the engaging component (1333A, 1333B) rotates.

7. The pumping and inserting mechanism (13A) of claim 6, **characterized in that** the triggering component (1331A, 1331B) comprises a first engaging structure (13311A), the output portion (1314A, 1314B) of the pumping assembly (131A, 131B, 131E, 131F, 131G, 131H, 131I) is disposed through the first engaging structure (13311A), and the engaging component (1333A, 1333B) comprises a second engaging structure (13332A) configured to engage with the first engaging structure (13311A).

8. The pumping and inserting mechanism (13A) of any of claims 3-7, **characterized in that** a rotating axis of the triggering component (1331A, 1331B) is coincided with a rotating axis of the output portion (1314A, 1314B) of the pumping assembly (131A, 131B, 131E, 131F, 131G, 131H, 131I), a rotating axis of the input portion (1313A, 1313B, 1313G, 1313H, 1313I) of the pumping assembly (131A, 131B, 131E, 131F, 131G, 131H, 131I) and a rotating axis of the engaging component (1333A, 1333B).

9. The pumping and inserting mechanism of claim 1, **characterized in that** the triggering component (1331C, 1331D) is coupled to the output portion (1314C, 1314D) of the pumping assembly (131C, 131D).

10. The pumping and inserting mechanism of any of claim 1 and 9, **characterized in that** a rotating axis of the triggering component (1331C, 1331D) is coincided with a rotating axis of the output portion (1314C, 1314D) of the pumping assembly (131C, 131D) and a rotating axis of the input portion of the pumping assembly (131C, 131D).

11. An automated injection system comprising:
a case (11A, 11F, 11G, 11H);
a reservoir (12A, 12F, 12H) mounted on the case (11A, 11F, 11G, 11H) internally or externally, or separated from the case (11A, 11F, 11G, 11H); and
**characterized by**:
the pumping and inserting mechanism (13A) of any of claims 1-10;
wherein the pumping assembly (131A, 131B, 131C, 131D, 131E, 131F, 131G, 131H, 1311) is at least partially mounted inside the case (11A, 11F, 11G, 11H), an inlet of the pumping assembly (131A, 131B, 131C, 131D, 131E, 131F, 131G, 131H, 131I) is communicated with the reservoir (12A, 12F, 12H), the driving assembly (132A, 132B, 132E, 132F, 132G, 132H, 132I) is mounted on the case (11A, 11F, 11G, 11H) internally or externally, the inserting assembly (133A, 133E, 133F) is at least partially mounted inside the case (11A, 11F, 11G, 11H), the inserting component (1332A, 1332B, 1332C, 1332D) is communicated with an outlet of the pumping assembly (131A, 131B, 131C, 131D, 131E, 131F, 131G, 131H, 1311).

## Amended claims

### Amended claims in accordance with Rule 137(2) EPC.

1. A pumping and inserting mechanism (13A) comprising:
a pumping assembly (131A, 131B, 131C, 131D, 131E, 131F, 131G, 131H, 1311) comprising an input portion (1313A, 1313B, 1313G, 1313H, 1313I) and an output portion (1314A, 1314B, 1314C, 1314D);
a driving assembly (132A, 132B, 132E, 132F, 132G, 132H, 132I) coupled to the input portion (1313A, 1313B, 1313G, 1313H, 1313I) of the pumping assembly (131A, 131B, 131C, 131D, 131E, 131F, 131G, 131H, 1311) and configured to drive a rotating movement of the input portion (1313A, 1313B, 1313G, 1313H, 1313I) of the pumping assembly (131A, 131B, 131C, 131D, 131E, 131F, 131G, 131H, 1311) to drive a rotating movement of the output portion (1314A, 1314B, 1314C, 1314D) of the pumping assembly (131A, 131B, 131C, 131D, 131E, 131F, 131G, 131H, 1311); and
an inserting assembly (133A, 133E, 133F) comprising a triggering component (1331A, 1331B, 1331C, 1331D) and an inserting component (1332A, 1332B, 1332C, 1332D), the triggering component (1331A, 1331B, 1331C, 1331D) being driven to rotate to trigger an inserting movement of the inserting component (1332A, 1332B, 1332C, 1332D) in response to the rotating movement of the output portion (1314A, 1314B, 1314C, 1314D) of the pumping assembly (131A, 131B, 131C, 131D, 131E, 131F, 131G, 131H, 1311);
**characterized in that** a rotating axis of the triggering component (1331A, 1331B, 1331C, 1331D) is coincided with a rotating axis of the output portion (1314A, 1314B, 1314C, 1314D) of the pumping assembly (131A, 131B, 131C, 131D, 131E, 131F, 131G, 131H, 1311) and a rotating axis of the input portion (1313A, 1313B, 1313G, 1313H, 1313I) of the pumping assembly (131A, 131B, 131C, 131D, 131E, 131F, 131G, 131H, 1311).

2. The pumping and inserting mechanism (13A) of claim 1, **characterized in that** the driving assembly (132A, 132B, 132E, 132F, 132G, 132H, 132I) comprises a driving component (1321A) and an adaptor (1322A), and the adaptor (1322A) is mounted between the driving component (1321A) and the input portion (1313A, 1313B, 1313G, 1313H, 1313I) of the pumping assembly (131A, 131B, 131C, 131D, 131E, 131F, 131G, 131H, 1311) and detachable from the input portion (1313A, 1313B, 1313G, 1313H, 13131) of the pumping assembly (131A, 131B, 131C, 131D, 131E, 131F, 131G, 131H, 1311).

3. The pumping and inserting mechanism (13A) any of claims 1 and 2, further **characterized by** an engaging component (1333A, 1333B) coupled to the output portion (1314A, 1314B) of the pumping assembly (131A, 131B, 131E, 131F, 131G, 131H, 1311) and configured to move from a disengaged state to an engaged state in response to the rotating movement of the output portion (1314A, 1314B, 1314G, 1314H, 1314I) of the pumping assembly (131A, 131B, 131E, 131F, 131G, 131H, 1311), the engaging component (1333A, 1333B) being disengaged from the triggering component (1331A, 1331B) for not driving the triggering component (1331A, 1331B) to rotate when the engaging component (1333A, 1333B) is not in the engaged state, and the engaging component (1333A, 1333B) being engaged with the triggering component (1331A, 1331B) for driving the triggering component (1331A, 1331B) to rotate when the engaging component (1333A, 1333B) is in the engaged state.

4. The pumping and inserting mechanism (13A) of claim 3, **characterized in that** the engaging component (1333A, 1333B) is a rotatable component.

5. The pumping and inserting mechanism (13A) of any of claims 3 and 4, **characterized in that** the engaging component (1333A, 1333B) is rotatable relative to the inserting assembly (133A, 133E, 133F), the engaging component (1333A, 1333B) comprises a first cooperating structure, the inserting assembly (133A, 133E, 133F) comprises a second cooperating structure configured to cooperate with the first cooperating structure to drive the engaging component (1333A, 1333B) to move from the disengaged state to the engaged state when the engaging component (1333A, 1333B) is driven to rotate in response to the rotating movement of the output portion (1314A, 1314B) of the pumping assembly (131A, 131B, 131E, 131F, 131G, 131H, 1311).

6. The pumping and inserting mechanism (13A) of any of claims 3-5, **characterized in that** the output portion (1314A, 1314B) of the pumping assembly (131A, 131B, 131E, 131F, 131G, 131H, 1311) is disposed through the triggering component (1331A, 1331B) and at least partially inserted into the engaging component (1333A, 1333B), and the engaging component (1333A, 1333B) is movable along an extending direction of the output portion (1314A, 1314B) of the pumping assembly (131A, 131B, 131E, 131F, 131G, 131H, 1311) when the engaging component (1333A, 1333B) rotates.

7. The pumping and inserting mechanism (13A) of claim 6, **characterized in that** the triggering component (1331A, 1331B) comprises a first engaging structure (13311A), the output portion (1314A, 1314B) of the pumping assembly (131A, 131B, 131E, 131F, 131G, 131H, 1311) is disposed through the first engaging structure (13311A), and the engaging component (1333A, 1333B) comprises a second engaging structure (13332A) configured to engage with the first engaging structure (13311A).

8. The pumping and inserting mechanism of claim 1, **characterized in that** the triggering component (1331C, 1331D) is coupled to the output portion (1314C, 1314D) of the pumping assembly (131C, 131D).

9. An automated injection system comprising:
a case (11A, 11F, 11G, 11H);
a reservoir (12A, 12F, 12H) mounted on the case (11A, 11F, 11G, 11H) internally or externally, or separated from the case (11A, 11F, 11G, 11H); and
**characterized by**:
the pumping and inserting mechanism (13A) of any of claims 1-8;
wherein the pumping assembly (131A, 131B, 131C, 131D, 131E, 131F, 131G, 131H, 1311) is at least partially mounted inside the case (11A, 11F, 11G, 11H), an inlet of the pumping assembly (131A, 131B, 131C, 131D, 131E, 131F, 131G, 131H, 1311) is communicated with the reservoir (12A, 12F, 12H), the driving assembly (132A, 132B, 132E, 132F, 132G, 132H, 132I) is mounted on the case (11A, 11F, 11G, 11H) internally or externally, the inserting assembly (133A, 133E, 133F) is at least partially mounted inside the case (11A, 11F, 11G, 11H), the inserting component (1332A, 1332B, 1332C, 1332D) is communicated with an outlet of the pumping assembly (131A, 131B, 131C, 131D, 131E, 131F, 131G, 131H, 1311).
